(19) Europäisches Patentamt
European Patent Office
Office européen des brevets

(11) Publication number: **0 211 863 B1**

(12) **EUROPEAN PATENT SPECIFICATION**

(45) Date of publication of patent specification: **30.12.92** (51) Int. Cl.$^5$: **C07J 9/00**

(21) Application number: **86900954.8**

(22) Date of filing: **15.01.86**

(86) International application number:
**PCT/US86/00093**

(87) International publication number:
**WO 86/04333 (31.07.86 86/17)**

(54) **VITAMIN D DERIVATIVES AND METHODS FOR PREPARING SAME.**

(30) Priority: **17.01.85 US 692151**
**30.05.85 US 739332**

(43) Date of publication of application:
**04.03.87 Bulletin 87/10**

(45) Publication of the grant of the patent:
**30.12.92 Bulletin 92/53**

(84) Designated Contracting States:
**BE CH DE FR GB IT LI NL**

(56) References cited:
**US-A- 3 880 894**

(73) Proprietor: **WISCONSIN ALUMNI RESEARCH FOUNDATION**
**614 North Walnut Street**
**Madison, WI 53705(US)**

(72) Inventor: **DeLUCA, Hector, F.**
**5130 Minocqua Crescent**
**Madison, WI 53705(US)**
Inventor: **IKEKAWA, Nobuo**
**2-21-5, Kichijojihigashi-cho**
**Musashinoshi Tokyo(JP)**
Inventor: **TANAKA, Yoko**
**72 Paxwood Road**
**Delmar, NY 12054(US)**
Inventor: **SCHNOES, Heinrich, K.**
**1806 Summit Avenue**
**Madison, WI 53705(US)**
Inventor: **OSTREM, Voula**
**4149 Manitou Way**
**Madison, WI 53711(US)**

(74) Representative: **Ellis-Jones, Patrick George Armine et al**
**J.A. KEMP & CO. 14 South Square Gray's Inn**
**London WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

### Technical Field

This invention relates to novel vitamin D derivatives.

More specifically, this invention relates to 26-homovitamins.

Still more specifically this invention relates to hydroxylated 26-homovitamins.

Vitamin D is known to regulate calcium and phosphorous metabolism in animals and humans and it has now been firmly established that the biological efficacy of vitamin D depends upon its metaboic conversion, in vivo, to hydroxylated derivatives. Thus vitamin $D_3$ is hydroxylated in vivo to 25-hydroxyvitamin $D_3$ in the liver which in turn is converted into 1$\alpha$,25-dihydroxyvitamin $D_3$ in the kidneys. It is the latter compound which is now recognized as being the circulating hormonal form of vitamin D.

Because of their biological activity in promoting calcium and phosphorous transport in the intestine and the mobilization and mineralization of bone these forms of vitamin D are important pharmaceutical products which are eminently suitable for use in the treatment of various bone disorders.

### Background Art

Vitamin D derivatives and their preparation and application are discussed in many references in the patent and other literature. For example, U.S. Patent No. 3,565,924 is directed to 25-hydroxycholecalciferol; U.S. Patent No. 3,697,559 is directed to 1,25-dihydroxycholecalciferol; U.S. Patent No. 3,741,996 is directed to 1$\alpha$-hydroxycholecalciferol; U.S. Patent No. 3,786,062 is directed to 22-dehydro-25-hydroxycholecalciferol; U.S. Patent No. 3,880,894 is directed to 1,25-dihydroxyergocalciferol; U.S. Patent No. 4,201,881 is directed to 24,24-difluoro-1$\alpha$,25-dihydroxycholecalciferol; U.S. Patent No. 4,196,133 is directed to 24,24-difluoro-1$\alpha$,25-dihydroxycholecalciferol.

### Disclosure of Invention

New derivatives of vitamin $D_3$ have now been found which express excellent vitamin D-like activity and which, for that reason, could readily serve as a substitute for vitamin $D_3$, as well as various of its derivatives, in known applications, such as, for example the treatment of various disease states manifesting calcium and phosphorous imbalance as hypoparathyroidism, osteodystrophy, osteomalacia and osteoporosis.

hese derivatives are 26-homovitamins and particularly 1$\alpha$,25-dihydroxy-22E-dehydro-26-homovitamin $D_3$ and 1$\alpha$,25-dihydroxy-26-homovitamin $D_3$.

The present invention provides a compound having the formula

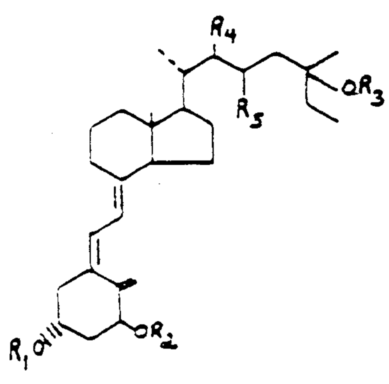

where $R_1$, $R_2$ and $R_3$ are independently hydrogen, an acyl group having from 1 to 4 carbon atoms or benzoyl, and $R_4$ and $R_5$ each represent a hydrogen atom or taken together form a carbon to carbon bond.

### Best Mode for Carrying Out the Invention

The compounds of this invention can be prepared in accordance with the process shown in the following schematic and process description. In the schematic and detailed description of the process like numbers identify like compounds.

## Schematic

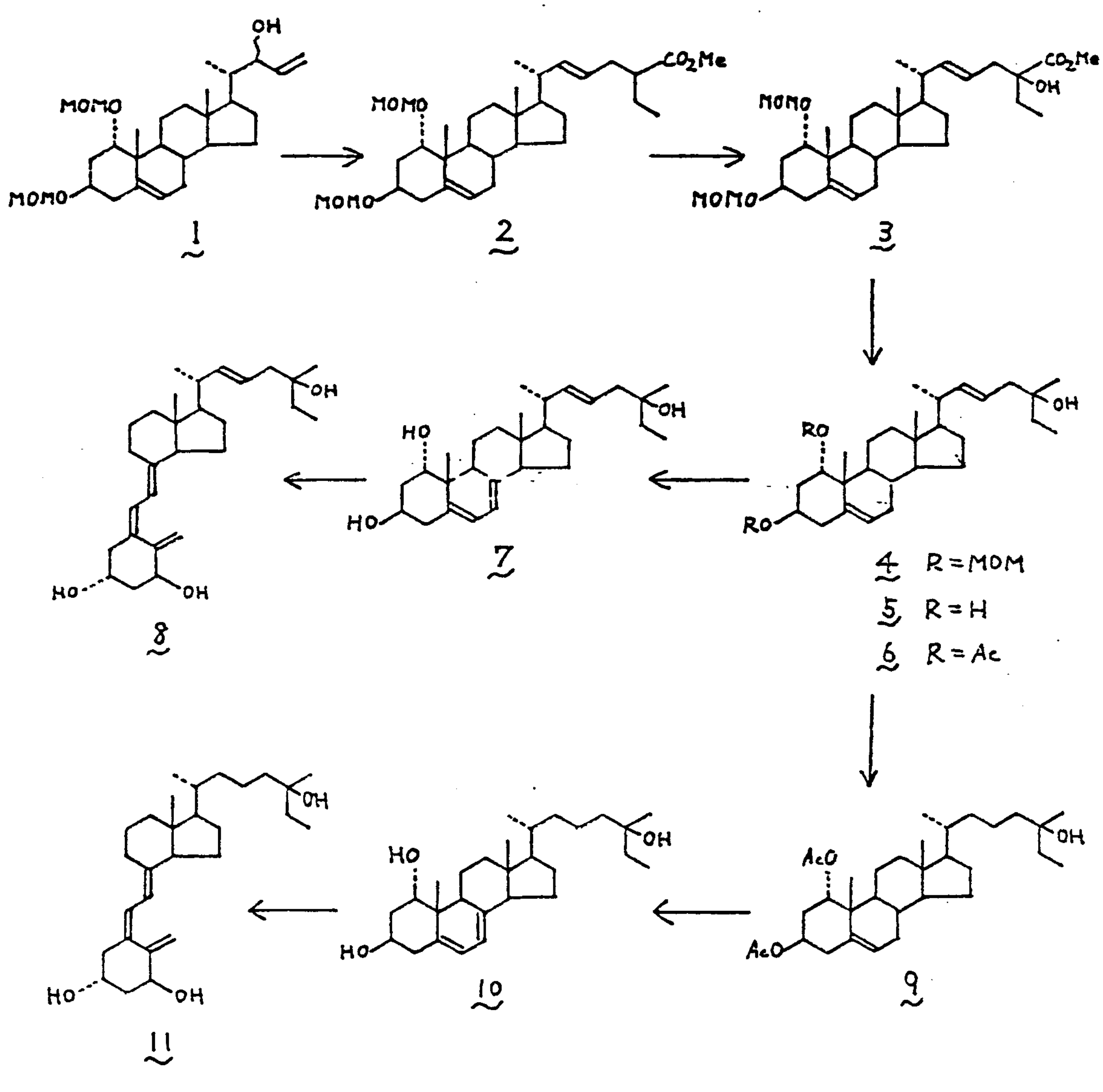

1
2
3
4 R=MOM
5 R=H
6 R=Ac
7
8
9
10
11

In accordance with the process of the present invention:

1a,3β-dimethoxymethoxy-23,24-dinorchol-5-en-22-al was reacted with vinyl magnesium bromide to provide the allyl alcohol (1). This alcohol was subjected to Claisen rearrangement by reacting it with trimethyl ortho-n-butylate and a catalytic amount of propionic acid to afford the ester (2) in good yield (97%). Compound (2) was converted to its enolate form by treatment with n-butyllithium and THF, which was then treated with oxygen and subsequently reduced with triethylphosphite to introduce an hydroxyl group at the C-25 position in the molecule. Then, the 27-ester (3) was converted to the alcohol (4) by successive treatment with lithium aluminum hydride to obtain the corresponding diol, followed by treatment with methanesulfonyl chloride and pyridine to provide the mesylate which, in turn was treated with lithium aluminum hydride.

Removal of the MOM group by treatment with acid gave (22E, 25ξ)-1α,3β,25-trihydroxy-26-homo-cholesta-5,22-diene (5) which was acetylated to provide the diacetate (6). Allylic bromination of 6) with N-bromosuccinimide and then with tetra-n-butylammonium fluoride gave the 5,7,22-triene (7) which was

irradiated and isomerized by heating to provide the (22E)-dehydroxy-26-homovitamin $D_3$ (8).

(25ξ)-1α,25-dihydroxy-26-homovitamin $D_3$ (11) was obtained by selectively hydrogenating the 5,22-diene (6) to provide the 5-ene (9), which was converted to the 5,7-diene (10) and then to the 26-homovitamin (11) as described above.

Detailed Description of Process

In the following detailed description of the process of this invention melting points were determined with a hot-stage microscope and were uncorrected. $^1$H-NMR spectra were taken with a Hitachi R-24A (60 MHz) in $CDCl_3$ with $Me_4Si$ as an internal standard, unless otherwise noted. Mass spectra were obtained with a Shimadzu QP-1000 mass spectrometer at 70 eV. UV spectra were obtained in ethanol solution with a Shimadzu UV-200 double beam spectrophotometer. Column chromatography was effected using silica gel (E. Merck, Kieselgel 60, 70-230 mesh). Preparative thin layer chromatography was carried out on precoated plates of silica gel (E. Merck, Kieselgel 60 $F_{254}$, 0.25 mm thickness). The usual work-up refers to dilution with water, extraction with an organic solvent, indicated in parenthesis, washing the extract to neutrality, drying over anhydrous magnesium sulphate, filtration, and removal of the solvent under reduced pressure. The following abbreviations were used; THP - tetrahydropyranyl; THF - tetrahydrofuran; ether - diethyl ether, MeOH - methanol, MOM - methoxymethyl, LDA - lithium diisopropyl amide. Temperatures are in ° centigrade.

(22E, 25ξ)-1α,3β-Dimethoxymethyloxy-26-homocholesta-5,22-dien-27-oic acid methyl ester (2)

A solution of the allylic alcohol (1) (390 mg, 0.844 mmol), trimethyl ortho-n-butylate (0.7 ml) and propionic acid (3 drops) in toluene (6 ml) was refluxed under argon for 2 h Removal of the solvent under reduced pressure gave a crude product, which was applied to a column of silica gel (20 g). Elution with hexane-ethyl acetate (5 : 1) gave the ester (2) (446 mg, 97%) as an oil. $^1$H-NMR δ:0.68 (3H, s, 18-$H_3$), 0.88 (3H, t, J = 7 Hz, -$CH_2CH_3$), 0.98 (3H, d, J = 6 Hz, 21-$H_3$) β.03 (3H, s, 19-$H_3$), 3.38 (3H, s, -$OCH_3$), 3.43 (3H, s, -$OCH_3$), 3.68 (3H, s, -$CO_2CH_3$), 3.76 (1H, m, 1β-H), 4.68 (2H, s, 3β-O-$CH_2$-O-), 4.69 (2H, ABq, J = 7 Hz, ΔAB = 11 Hz, 1α-O-$CH_2$-O), 5.27 (2H, m, 22-H and 23-H), and 5.56 (1H, m, 6-H).

(22E,25ξ)-1α,3β-Dimethoxymethyloxy-25-hydroxy-26-homocholesta-5,22-dien-27-oic acid methyl ester (3)

To a solution of LDA (prepared with diisopropylamine (0.13 ml, 0.929 mmol), 1.56 M n-butyllithium (0.59 ml) and THF (2 ml), the ester (2) (437 mg, 0.800 mmol) in THF (5 ml) was added and the mixture was stirred under argon at -78°C for 30 min. Oxygen was bubbled into this solution and then triethylphosphite (0.14 ml, 0.817 mmol) was added. The usual work-up (ether for extraction) gave a crude product, which was applied to a column of silica gel (25 g). Elution with hexane-ethyl acetate (5 : 1) provided the hydroxy ester (3) (303 mg, 67%) as an oil. $^1$H-NMR δ:0.68 (3H, s, 18-$H_3$), 0.85 (3H, t, J = 7 Hz, -$CH_2CH_3$), 0.98 (3H, d, J = 6 Hz, 21-$H_3$), 1.02 (3H, s, 19-$H_3$), 3.08 (1H, bs, $W_{1/2}$ = 3 Hz, -OH), 3.38 (3H, s, -$OCH_3$), 3.42 (3H, s, -$OCH_3$), 3.76 (3H, s, -$CO_2CH_3$), 4.68 (2H, s, 3β-O-$CH_2$-O-), 4.68 (2H, ABq, J = 7Hz, ΔAB = 11 Hz, 1α-O-$CH_2$-O-), 5.32 (2H, m, 22-H and 23-H), 5.55 (1H, m, 6-H).

(22E,25ξ)-1α,3β-Dimethoxymethyloxy-25-hydroxy-26-homocholesta-5,22-diene (4)

To a solution of the hydroxyester (3) (294 mg, 0.539 mmol) in THF (5 ml), lithium aluminum hydride (20 mg, 0.526 mmol) was added and this mixture was stirred at room temperature for 30 min. The usual work-up (ether for extraction) gave a crude diol. This was treated with methanesulfonyl chloride (0.04 ml, 0.517 mmol) and pyridine (1.5 ml) at room temperature for 30 min. The usual work-up (ether for extraction) gave a crude mesylate. To a solution of the crude mesylate in THF (5 ml), lithium aluminum hydride (20 mg, 0.526 mmol) was added and the mixture was refluxed for 30 min. The usual work-up (ether for extraction) gave a crude product, which was applied to a column of silica gel (20 g). Elution with hexane-ethyl acetate (5 : 1) provided the alcohol (4) (190 mg, 70%) as an oil. $^1$H-NMR δ:0.71 (3H, s, 18-$H_3$), 0.90 (3H, t, J = 7 Hz, -$CH_2CH_3$), 1.03 (3H, d, J = 6 Hz, 21-$H_3$), 1.03 (3H, s, 19-$H_3$), 1.12 (3H, s, 27-$H_3$), 3.36 (3H, s, -$OCH_3$) 3.40 (3H, s, -$OCH_3$), 3.74 (1H, m, 1β-H), 4.66 (2H, s, 3β-O-$CH_2$-O-), 4.67 (2H, ABq, J = 7 Hz, ΔAB = 11 Hz, 1α-O-$CH_2$-O-), 5.35 (2H, m, 22-H and 23-H) and 5.54 (1H, m, 6-H).

(22E,25ξ)-1α,3β,25-Trihydroxy-26-homocholesta-5,22-diene (5)

A solution of the dimethoxymethyl ester (4) 181 mg, 0.349 mmol) in THF (5 ml) was treated with 6N HCl (1 ml) at 50 ° C for 1.5 h. The usual work-up (ethyl acetate for extraction) gave a crude product, which was applied to a column of silica gel (15 g). Elution with hexane-ethyl acetate (1 : 2) provided the triol (5) (147 mg, 98%), m.p. 85-87 ° C (hexane-dichloromethane). $^1$H-NMR δ:0.69 (3H, s, 18-$H_3$), 0.89 (3H, t, J = 7 Hz, -$CH_2CH_3$), 1.02 (3H, s, 19-$H_3$), 1.13 (3H, s, 27-$H_3$), 3.85 (1H, m, 1β-H), 3.98 (1H, m, 3α-H), 5.40 (2H, m, 22-H and 23-H), and 5.60 (1H, m, 6-H).

(22E,25ξ)-1α,3β-Diacetoxy-25-hydroxy-26-homocholesta-5,22-diene (6)

A solution of the triol (5) (100 mg, 0.233 mmol) in pyridine (1 ml) was treated with acetic anhydride (1 ml) at room temperature for 15 h. The usual work-up (ethyl acetate for extraction) gave a crude product, which was applied to a column of silica gel (10 g). Elution with hexane-ethyl acetate (5 : 1) provided the diacetate (6) (101 mg, 85%) as an amorphous solid. $^1$H-NMR δ:0.68 (3H, s, 18-$H_3$), 0.88 (3H, t, J = 7 Hz, -$CH_2CH_3$), 0.98 (3H, d, J = 6Hz, 21-$H_3$), 1.08 (3H, s, 19-$H_3$), 1.12 (3H, s, 27-$H_3$), 2.03 (3H, s, acetyl), 2.06 (3H, s, acetyl), 4.98 (1H, m, 3α-H), 5.06 (1H, m, 1β-H), 5.37 (2H, m, 22-H and 23-H), and 5.53 (1H, m, 6-H).

(22E,25ξ)-1α,3β,25-Trihydroxy-26-homocholesta-5,7,22-triene (7)

A solution of the 5,22-diene (6) (38 mg, 0.0739 mmol) and N-bromosuccinimide (19 mg, 0.107 mmol) in carbontetrachloride (3 ml) was refluxed under argon for 20 min. After cooling to 0 ° C, the resulting precipitate was filtered off. The filtrate was concentrated below 40 ° C to leave the residue. The THF (5 ml) solution of this residue was treated with a catalytic amount of tetra-n-butyl ammonium bromide at room temperature for 50 min. Then, the mixture was treated with a solution of tetra-n-butylammonium fluoride in THF (0.3 ml, 0.3 mmol) at room temperature for 30 min. The usual work-up (ethyl acetate for extraction) gave a crude triene. This triene in THF (5 ml) was treated with 5% KOH-MeOH (4 ml) at room temperature for 14 h. The usual work-up (ethyl acetate for extraction) gave a crude product, which was submitted to preparative thin layer chromatography (benzene-ethyl acetate, 1 : 1, developed six times). The band of Rf value 0.45 was scraped off and eluted with ethyl acetate. Removal of the solvent provided the 5,7,22-triene (7) (8.7 mg, 40%).

$$UV\ \lambda^{EtOH}_{max}:$$

293, 282, 271 nm.

(22E,25ξ)-1α,25-Dihydroxy-22-dehydro-26-homovitamin $D_3$ (8)

A solution of the triene (7) (4.4 mg, 0.0103 mmol) in benzene (90 ml) and ethanol (40 ml) was irradiated with a medium pressure mercury lamp through a Vycor® filter at 0 ° C under argon for 2.5 min. The reaction mixture was refluxed under argon for 1 h. Removal of the solvent under reduced pressure gave a crude product, which was submitted to preparative thin layer chromatography (benzene-ethyl acetate, 1 : 1, developed six times). The band of Rf value 0.49 was scraped off and eluted with ethyl acetate. Removal of the solvent provided the vitamin $D_3$ analogue (8) (0.91 mg, 21%).

$$UV\ \lambda^{EtOH}_{max}:$$

265 nm,

$$\lambda^{EtOH}_{min}:$$

282 nm. MS m/z: 428 ($M^+$), 410, 392, 374, 338, 320, 287, 269, 251, 141, 134, 123, 73. $^1$H-NMR (400 MHz) δ : 0.56 (3H, s, 18-$H_3$), 0.91 (3H, t, J = 7.6 Hz, -$CH_2CH_3$), 1.04 (3H, d, J = 6.8 Hz, 21-$H_3$), 1.13 (3H, s, 27-$H_3$), 4.23 (1H, m, $W_{1/2}$ = 18.4 Hz, 3α-H), 4.43 (1H, m, $W_{1/2}$ = 16.9 Hz, 1β-H), 5.00 (1H, bs, $W_{1/2}$ = 3.2 Hz, 19-H), 5.32 (1H, bs, $W_{1/2}$ = 3.2 Hz, 19-H), 5.37 (2H, m, 22-H and 23-H), 6.02 (1H, d, J - 11.5 Hz, 7-H), and

6.38 (1H, d, J = 11.5 Hz, 6-H).

(25ξ)-1α,3β-Diacetoxy-25-hydroxy-26-homocholest-5-ene (9)

A mixture of the 5,22-diene (6) (35 mg, 0.0681 mmol) and 10% Pd-C (4 mg) in ethyl acetate (4 ml) was stirred at room temperature under hydrogen for 3 h. The Pd catalyst was filtered off and the filtrate was concentrated to leave the residue, which was submitted to preparative thin layer chromatography (hexane-ethyl acetate, 2 : 1, developed once). The band of Rf value 0.46 was scraped off. Elution with ethyl acetate provided the 5-ene (9) (30 mg, 85%) as an amorphous solid. $^1$H-NMR δ : 0.66 (3H, s, 18-$H_3$), 0.88 (3H, t, J = 7 Hz, -$CH_2CH_3$), 1.08 (3H, s, 19-$H_3$), 1.12 (3H, s, 27-$H_3$), 2.02 (3H, s, acetyl), 2.04 (3H, s, acetyl), 4.97 (1H, m, 3α-H), 5.04 (1H, m, 1β-H), and 5.51 (1H, m, 6-H).

(25ξ)-1α,3β,25-Trihydroxy-26-homocholesta-5,7-diene (10)

The 5-ene (22 mg, 0.0426 mmol) was converted, as described for (7), to the 5,7-diene 10 (6.7 mg, 37%).

$$UV\ \lambda^{EtOH}_{max}:$$

293, 282, 271 nm.

(25ξ)-1α,25-Dihydroxy-26-homovitamin $D_3$ (11)

The diene (10) (4.8 mg, 0.0112 mmol) was converted, as described for (8), to the vitamin $D_3$ analogue (11) (1.3 mg, 27%).

$$UV\ \lambda^{EtOH}_{max}:$$

265 nm,

$$\lambda^{EtOH}_{min}:$$

228 nm. MS m/z: 430 ($M^+$), 412, 394, 379, 376, 287, 269, 251, 152, 134, 116, 73, 55.

If desired, the compounds of this invention can be readily obtained in crystalline form by crystallization from suitable solvents, e.g. hexane, ethers, alcohols, or mixture thereof as will be apparent to those skilled in the art.

## Biological Activity

### Bone calcium mobilization activity of 1α,25-$(OH)_2$-26-homo-$D_3$ compounds

Male weanling rats were purchased from Holtzman Co., Madison, Wis. and fed ad libitum a low calcium, vitamin D deficient diet as described by Suda et al (J. Nutrition 100: 1049, 1970) and water for 3 weeks. The rats were then divided into 4 groups of 6 each and were intrajugularly given respectively 650 pmole of either 1α,25-$(OH)_2$-26-homo-$D_3$, 1α,25-$(OH)_2$-(22E)$\Delta^{22}$-26-homo-$D_3$ or 1α,25-$(OH)_2D_3$ dissolved in 0.05 ml of 95% ethanol 7 h prior to sacrifice. The rats in the control group were given 0.05 ml of 95% ethanol 7 h prior to sacrifice. The rats in the control group were given 0.05 ml of ethanol vehicle in the same manner. They were killed by decapitation, the blood was collected and centrifuged to obtain serum. Serum calcium concentration was determined with an atomic absorption spectrophotometer (Perkin-Elmer Model 214) in presence of 0.1% lanthanum chloride. Results are shown in the table below:

Table 1

| Compound Administered | Serum Calcium Concentration (mg/100 ml) |
|---|---|
| ethanol | 3.4 ± 0.3* a) |
| $1\alpha$,25-$(OH)_2$-26-homo-$D_3$ | 4.6 ± 0.2 b) |
| $1\alpha$,25-$(OH)_2$-(22E)$\Delta^{22}$-26-homo-$D_3$ | 4.6 ± 0.3 b) |
| $1\alpha$,25-$(OH)_2D_3$ | 4.5 ± 0.2 b) |

*standard deviation from the mean
b) is significantly different from a) P 0.001

It can be concluded from the foregoing data that in the vitamin D responsive systems of vitamin D-deficient animals the compounds of this invention exhibited the same activity as $1\alpha$,25-hydroxyvitamin $D_3$, the circulating hormonal form of the vitamin.

The compounds of this invention may be readily administered for purposes of their calcemic activity in sterile parenteral solutions by injection or intravenously or by alimentary canal in the form of oral dosages, or by spppository or even transcutaneously. Doses of from 0.1 $\mu$g to 2.5 $\mu$g per day are generally effective in obtaining the physiological calcium balance responses characteristic of vitamin D-like activity with maintenance dosage of from 0.1 $\mu$g to 0.5 $\mu$g being suitable.

It has recently been discovered that $1\alpha$,25-dihydroxy-vitamin $D_3$ ($1\alpha$,25-$(OH)_2D_3$) and its structural analog $1\alpha$-hydroxyvitamin $D_3$ ($1\alpha$-OH-$D_3$), in addition to their well-established calcemic action referred to above, also express potent anti-cancer activity. Specifically, it was shown that the above-named compounds were effective in causing differentiation of malignant human cells, such as leukemia cells in culture, to non-malignant macrophages, and the anti-cancer activity on cells in vitro could be correlated with beneficial effects in vivo by showing that the administration of these compounds extended the life span of leukemic mice (compared to controls) and markedly improved the condition of human leukemia patients. Based on these observations, $1\alpha$-hydroxylated vitamin D compounds have been proposed as therapeutic agents for the treatment of leukemoid diseases (Suda et al., U.S. Patent No. 4,391,802).

Although these known $1\alpha$-hydroxyvitamin D compounds tested by Suda et al. (supra), namely $1\alpha$-hydroxyvitamin $D_3$ ($1\alpha$-OH-$D_3$) and $1\alpha$,25-dihydroxyvitamin $D_3$ ($1\alpha$,25-$(OH)_2D_3$), are indeed highly effective in causing differentiation of leukemic cells, a serious disadvantage to their use as antileukemic agents is the inherent, and hence unavoidable high calcemic activity of these substances. Thus, $1\alpha$,25-$(OH)_2D_3$, the most potent vitamin-derived antileukemic agent known thus far, is also the most potent calcemic agent, and the antileukemic potency of $1\alpha$-OH-$D_3$ is likewise correlated with high calcemic activity. The administration of these compounds, at the dosage level where they are effective as antileukemic drugs (e.g. 1 ug/day as specified in the examples of the Suda et al. patent), would necessarily produce elevated, potentially excessive, calcium levels with attendant serious medical complications, particularly in patients already suffering from debilitating disease. Because of the high intrinsic potency of the known $1\alpha$-hydroxyvitamin D compounds in raising calcium levels, their use as antileukemic agents nay be precluded.

A preferred method of treatment of malignant disease states clearly would be the administration of compounds characterized by a high antileukemic to calcemic activity ratio, that is, of compounds exhibiting an enhanced potency in causing differentiation of leukemic cells as compared to their potency in raising serum calcium levels.

The compounds of this invention are also preferentially active in inducing the differentiation of malignant cells to non-malignant cells, i.e. in antineoplastic activity as measured by leukemia cell differentiation, while being no more active than $1\alpha$,25-dihydroxyvitamin $D_3$ in their effect on calcium metabolism. Because of this unique and unexpected combination of properties, the novel side-chain homovitamin D compounds of this invention represent superior and preferred agents for the treatment of leukemias and other neoplastic diseases.

When administered to human promyelocytic leukemia cells (HL-60 cells) grown in culture, the side-chain homovitamin D compounds of this invention induce the differentiation of these cells to macrophages (monocytes). In several standard assays for measuring differentiation activity, these compounds were shown to be more effective than $1\alpha$,25-$(OH)_2D_3$, the most active vitamin D derivative known thus far. These assays were performed as follows:

Assay of homovitamin D compounds for differentiation activity.

The human promyelocytic leukemia cell line (HL-60) was maintained in suspension culture in RPMI 1640 medium (Gibco, Grand Island, NY) supplemented with 10% (v/v) heat inactivated fetal calf serum, 100 ug/ml penicillin, 100 μg/ml streptomycin and 0.25 μg/ml fungizone. Cells were cultured in a humidified atmosphere with 5% $CO_2$. Cell viability was assessed by standard assays, e.g. trypan blue exclusion. Morphological evaluations were done on Wright stained slide preparations.

Cells were seeded at 1.5 - 2 x $10^5$ cells/ml in 10 ml of medium in tissue culture dishes. After 20 h, duplicate dishes were then treated with each of the test compounds at various concentrations as indicated in the tables below. The test compounds were added as solutions in 100% ethanol so that the total ethanol concentration in each culture dish did not exceed 0.2%. Control cultures were treated with the same concentration of ethanol. After four days (96 hr) of incubation with test compounds, the cells were harvested from these culture dishes and cell number and viability were determined. The extent of differentiation induced by the tested vitamin D derivatives was expressed as the percentage of cells that exhibit functional and enzymatic markers characteristic of monocytes. The two markers assayed were a) the ability of the cells to phagocytize dead yeast, and b) the ability of the cells to produce superoxide (reduce nitroblue tetrazolium) when stimulated with phorbol esters.

a) Phagocytosis Assay for Differentiation Activity: The harvested cells were resuspended in RPMI medium containing 20% AB serum and 20% fetal calf serum, to give a preparation containing 2 x $10^6$ cells/ml. To 0.5 ml ($10^6$ cells) of the above cell suspension was then added 0.5 ml of a suspension (in phosphate-buffered saline) of heat-killed *Saccharomyces cerevisiae* cells (1 x $10^8$ cells) which had been stained with trypan blue. After incubation of this mixture for 1 hr at 37°C, the number of phagocytic cells was counted (as determined by the trypan blue stained yeast appearing intracellularly) and expressed as a percent of the total viable cells present. This "% phagocytic cells" indicates the percent of differentiation induced by the test compounds. Results are summarized in Table 2 below.

Table 2

Percent phagocytic (differentiated) cells produced in HL-60 cell cultures treated with vitamin D compounds at various concentrations

| Compound Administered | Concentration (moles/liter) | | | | | | |
|---|---|---|---|---|---|---|---|
| | 0[(a,b)] | $3x10^{-10}$ | $5x10^{-10}$ | $1x10^{-9}$[(b)] | $1x10^{-8}$[(b)] | $1x10^{-7}$[(b)] | $3x10^{-7}$ |
| $1,25-(OH)_2D_3$ | $10 \pm 1.5$ | 17 | 23 | $28 \pm 4$ | $47 \pm 1$ | $67 \pm 6$ | 69 |
| homo-cpd I* | $10 \pm 1.5$ | 28 | 38 | $44 \pm 5$ | $72 \pm 2$ | $76 \pm 3$ | 77 |
| homo-cpd II** | $10 \pm 1.5$ | 22 | 42 | $48 \pm 6$ | $70 \pm 0$ | $78 \pm 4$ | 83 |

[a]Control level; cell cultures were treated with solvent ethanol only.

[b]Results tabulated in these columns represent the mean $\pm$ SEM of three different experiments, each done in duplicate.

*1α,25-dihydroxy-26-homovitamin $D_3$

**1α,25-dihydroxy-22E-dehydro-26-homovitamin $D_3$

The results in Table 2 show that the homo compounds are significantly more potent than $1,25-(OH)_2D_3$. At all concentrations, the homo compounds achieve a greater degree of differentiation of the leukemia cells than $1\alpha,25-(OH)_2D_3$, the most active compound known thus far. For example, at a concentration of $10^{-8}$ molar the homo compounds achieve a differentiation of 70%, whereas $1,25-(OH)_2D_3$ at the same concentration gives only about 47% differentiated cells. To achieve 50% differentiation requires a concentration of 1 x $10^{-9}$ M of the homo compounds, but about 1 x $10^{-8}$ M of $1\alpha,25-(OH)_2D_3$, i.e. a difference in potency of about 10-fold.

b) NBT-Reduction Assay for Differentiation: This assay depends on the ability of monocyte-like leukemia cells to reduce the nitroblue tetrazolium (NBT) reagent to a black-blue precipitate (formazan) when stimulated by phorbol esters. The assay was performed according to the general procedure given by Yen et al (J. Cellular Physiol. 118, 277 (1984)). The cells were harvested as above and then suspended in RPMI medium; to 0.2 ml of this suspension (containing about 1.4 x $10^6$ cells/ml) was added 0.2 ml of the nitroblue tetrazolium (NBT) reagent. (The NBT reagent was prepared by mixing a solution containing 50 mg of nitroblue tetrazolium in 50 ml of phosphate-buffered saline with 10 microliters of an acetone/water (1:1) solution containing 0.5 mg/ml of 4β-phorbol- 12-myristate-13-acetate). After standing

in a water bath for 30 min, the differentiated cells (i.e. the cells showing formazan blue deposits indicative of NBT reduction) were counted with a hemocytometer and expressed as the percent of total viable cells present. The results of this assay are shown in Table 3 below.

Table 3

Percent of cells in HL-60 cell cultures exhibiting nitroblue tetrazolium (NBT) reduction activity after treatment with Vitamin D Compounds at various concentrations

| Compound Administered | Concentration (moles/liter) | | | | | | |
|---|---|---|---|---|---|---|---|
| | 0[a,b] | $3x10^{-10}$ | $5x10^{-10}$ | $1x10^{-9}$(b) | $1x10^{-8}$(b) | $1x10^{-7}$(b) | $3x10^{-7}$ |
| 1,25-$(OH)_2D_3$ | 10±1.5 | 15 | 27 | 31±4 | 45±4 | 69±7 | 65 |
| homo-cpd I* | 10±1.5 | 27 | 41 | 47±7 | 72±5 | 79±2 | 78 |
| homo-cpd II** | 10±1.5 | 22 | 44 | 49±4 | 70±2 | 79±5 | 80 |

a Control level; cell cultures treated with solvent ethanol only.

b Data represent the mean ± SEM of three separate experiments, each assayed in duplicate.

*1α,25-dihydroxy-26-homovitamin $D_3$

**1α,25-dihydroxy-22E-dehydro-26-homovitamin $D_3$

The results shown in Table 3 again establish that the homo compounds tested are more active than 1α,25-$(OH)_2D_3$ in inducing the differentiation of human myeloid leukemia cells to normal cells, in vitro. To achieve 60% differentiation of the leukemic cells as measured by this NBT reduction assay, requires a concentration of 2 x $10^{-9}$ M of the homo compounds; to achieve the same degree of differentiation with 1α,25-$(OH)_2D_3$ requires a concentration of 3.5 x $10^{-8}$ M, a 17-fold difference in potency.

Thus, both of the above assays confirm the high potency of the homovitamin D compounds in inducing the differentiation of leukemic cells. In addition, the above results show that in this differentiation activity these homovitamin D compounds are significantly more potent than 1α,25-$(OH)_2D_3$.

Since this differentiating activity is expressed in the case of human leukemia cells (HL-60), it is clear that these novel homovitamin D compounds can be used effectively against leukemias in human subjects. At the same time, these compounds do not exhibit enhanced calcemic activity, but are about as active as

1α,25-$(OH)_2D_3$. Thus, these homovitamin D compounds are characterized by a high antineoplastic to calcemic activity ratio. By virtue of this novel and desirable biological property, these side-chain homo compounds would function as superior therapeutic agents for the treatment of malignant diseases.

For the treatment of human leukemia, the homovitamin D compounds of this invention are administered to subjects in dosages sufficient to induce the differentiation of leukemic cells to macrophages. Suitable dosage amounts are from 0.2 μg to 5 μg per day, it being understood that dosages can be adjusted according to the severity of the disease or the reponse or the condition of subject as is well-understood in the art.

For treatment purposes, dosage forms of the compounds can be prepared by ccmbining them with a non-toxic pharmaceutically acceptable carrier as is well known in the art. Such carriers may be either solid or liquid such as, for example, corn starch, lactose, sucrose, peanut oil, olive oil, sesame oil and water. If a solid carrier is used the dosage forms of the compounds of the invention may be tablets, capsules, powders, troches or lozenges. If a liquid carrier is used, soft gelatin capsules, or syrup or liquid suspensions, emulsions or solutions may be the dosage form. The dosage forms may also contain adjuvants, such as preserving, stabilizing, wetting or emulsifying agents and solution promoters. They may also contain other therapeutically valuable substances.

It should be understood that although dosage ranges are given the particular dose to be administered to a host will depend upon the specific disease state being treated, the end results being sought in a particular case, the physical size of the host, as well as other factors known to those skilled in the art in the therapeutic use of such medicinal agents.

The compounds can be advantageously administered by injection, or by intravenous infusion of suitable sterile solutions, or in the form of oral doses via the alimentary canal.

**Claims**

**1.** A compound having the formula

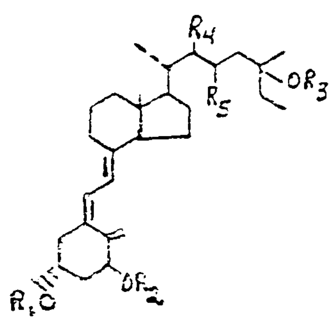

where $R_1$, $R_2$ and $R_3$ are independently hydrogen, an acyl group having from 1 to 4 carbon atoms or benzoyl, and $R_4$ and $R_5$ each represent a hydrogen atom or taken together form a carbon to carbon bond.

**2.** A compound according to claim 1 wherein $R_1$, $R_2$ and $R_3$ are hydrogen and $R_4$ and $R_5$ are hydrogen atoms.

**3.** The compound of claim 2 in crystalline form.

**4.** A compound according to claim 1 wherein $R_1$, $R_2$ and $R_3$ are hydrogen and $R_4$ and $R_5$ together represent a carbon to carbon bond.

**5.** The compound of claim 4 in crystalline form.

**6.** A compound according to claim 4 or 5 wherein the $\Delta^{22}$ bond is in the E configuration.

**7.** A pharmaceutical composition which comprises at least one compound as claimed in any one of the preceding claims and a pharmaceutically acceptable excipient.

**8.** A compound having the formula

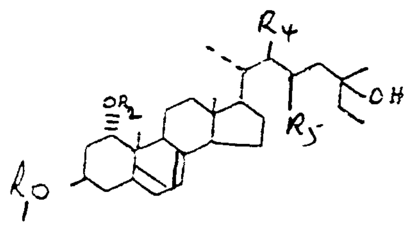

where $R_1$, $R_2$ and $R_3$ are independently hydrogen, an acyl group having from 1 to 4 carbon atoms or benzoyl and $R_4$ and $R_5$ represent hydrogen atoms or taken together form a carbon to carbon bond.

**9.** A compound of formula:

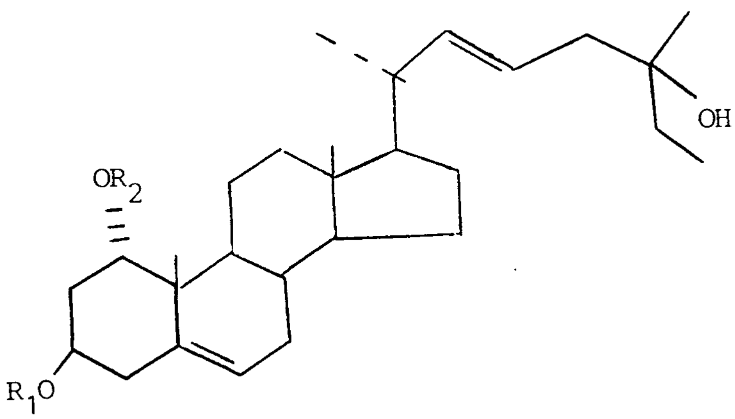

wherein $R_1$ and $R_2$ are independently hydrogen, an acyl group having from 1 to 4 carbon atoms, benzoyl or methoxymethyl.

**10.** A compound of formula:

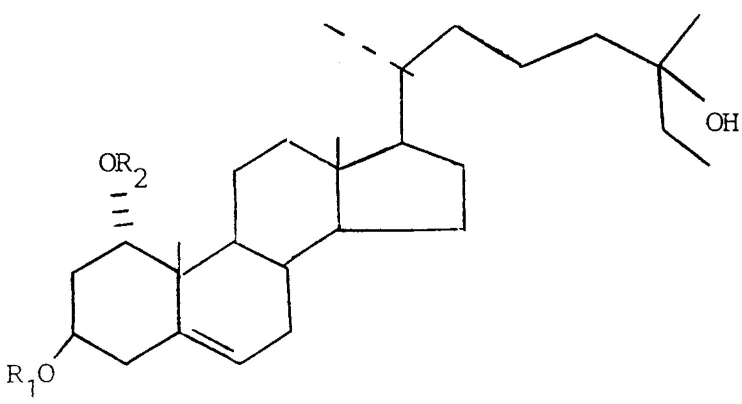

wherein $R_1$ and $R_2$ are both acetyl.

**11.** A compound as claimed in any one of claims 1 to 6 for use in the induction and enhancement of cell differentiation in malignant cells.

**Patentansprüche**

**1.** Verbindung der Formel

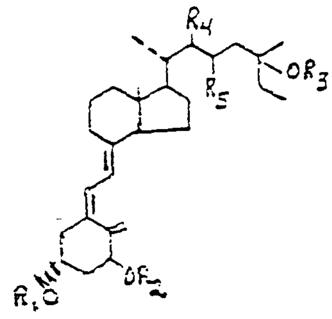

worin $R_1$, $R_2$ und $R_3$ unabhängig voneinander Wasserstoff, eine Acylgruppe mit 1 bis 4 Kohlenstoffatomen oder Benzoyl bedeuten und $R_4$ und $R_5$ jeweils ein Wasserstoffatom darstellen oder gemeinsam eine Kohlenstoff-Kohlenstoffbindung bilden.

**2.** Verbindung nach Anspruch 1 worin $R_1$, $R_2$ und $R_3$ Wasserstoff und $R_4$ und $R_5$ Wasserstoffatome bedeuten.

**3.** Verbindung nach Anspruch 2 in kristalliner Form.

**4.** Verbindung nach Anspruch 1, worin $R_1$, $R_2$ und $R_3$ Wasserstoff bedeuten und $R_4$ und $R_5$ zusammen eine Kohlenstoff-Kohlenstoffbindung darstellen.

**5.** Verbindung nach Anspruch 4 in kristalliner Form.

**6.** Verbindung nach Anspruch 4 oder 5, worin die $\blacktriangle^{22}$ Bindung sich in der E-Konfiguration befindet.

**7.** Pharmazeutische Zusammensetzung, die mindestens eine Verbindung wie in einem der vorhergehenden Ansprüche beansprucht und einen pharmazeutisch verträglichen Träger enthalten.

**8.** Verbindung der Formel

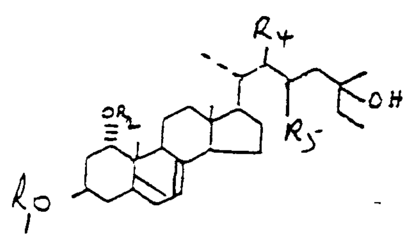

worin $R_1$, $R_2$ und $R_3$ unabhängig voneinander Wasserstoff, eine Acylgruppe mit 1 bis 4 Kohlenstoffatomen oder Benzoyl bedeuten und $R_4$ und $R_5$ Wasserstoffatome oder gemeinsam eine Kohlenstoff-Kohlenstoffbindungdarstellen.

**9.** Verbindung der Formel

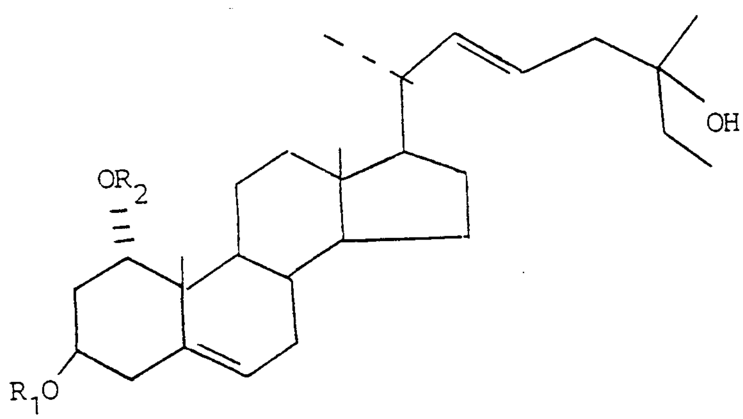

worin $R_1$ und $R_2$ unabhängig voneinander Wasserstoff, eine Acylgruppe mit 1 bis 4 Kohlenstoffatomen, Benzoyl oder Methoxymethyl bedeuten.

**10.** Verbindung der Formel

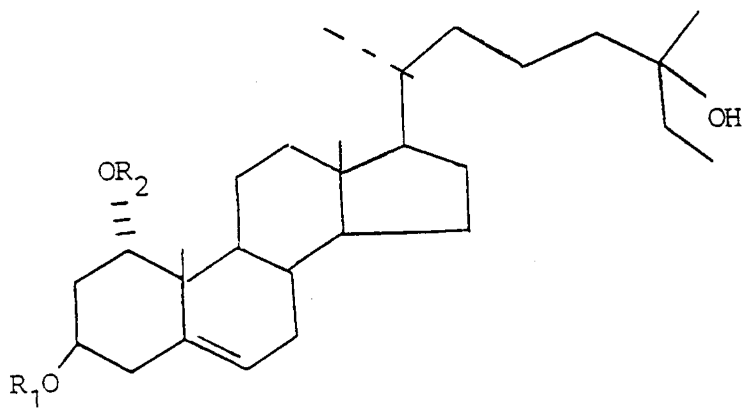

worin $R_1$ und $R_2$ beide Acetyl bedeuten.

**11.** Verbindung gemäß einem der Ansprüche 1 bis 6 zur Verwendung bei der Induzierung und Steigerung der Zelldifferenzierung in malignen Zellen.

**Revendications**

**1.** Composé présentant la formule

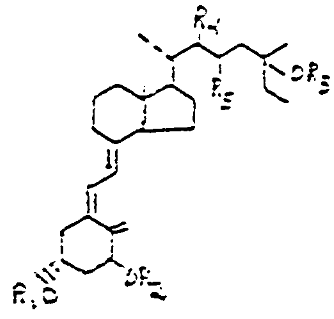

dans laquelle $R_1$, $R_2$ et $R_3$ représentent chacun, indépendamment, un atome d'hydrogène, un groupe acyle comportant de 1 à 4 atomes de carbone ou un groupe benzoyle, et $R_4$ et $R_5$ représentent chacun

un atome d'hydrogène, ou forment conjointement une liaison carbone-carbone.

**2.** Composé conforme à la revendication 1, dans lequel $R_1$, $R_2$ et $R_3$ représentent des atomes d'hydrogène et $R_4$ et $R_5$ représentent des atomes d'hydrogène.

**3.** Composé conforme à la revendication 2, sous forme cristalline.

**4.** Composé conforme à la revendication 1, dans lequel $R_1$, $R_2$ et $R_3$ représentent des atomes d'hydrogène, et $R_4$ et $R_5$ représentent conjointement une liaison carbone-carbone.

**5.** Composé conforme à la revendication 4, sous forme cristalline.

**6.** Composé conforme à la revendication 4 ou 5, dans lequel la liaison $\Delta^{22}$ se trouve en configuration E.

**7.** Composition pharmaceutique qui contient au moins un composé conforme à l'une quelconque des revendications précédentes et un excipient pharmaceutiquement acceptable.

**8.** Composé présentant la formule

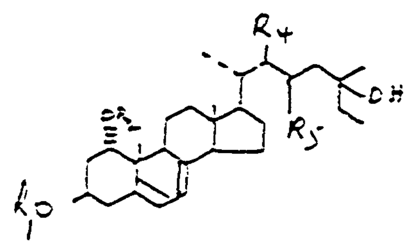

dans laquelle $R_1$, $R_2$ et $R_3$ représentent chacun, indépendamment, un atome d'hydrogène, un groupe acyle comportant de 1 à 4 atomes de carbone ou un groupe benzoyle, et $R_4$ et $R_5$ représentent chacun un atome d'hydrogène, ou forment conjointement une liaison carbone-carbone.

**9.** Composé de formule

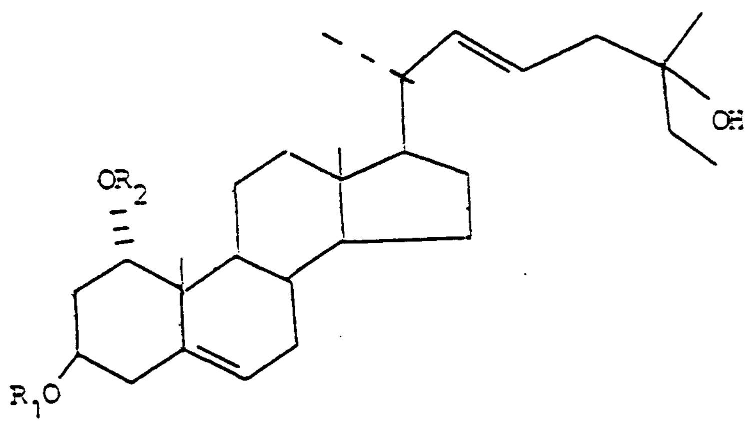

dans laquelle $R_1$ et $R_2$ représentent chacun indépendamment un atome d'hydrogène, un groupe acyle comportant de 1 à 4 atomes de carbone, un groupe benzoyle ou un groupe méthoxyméthyle.

**10.** Composé de formule

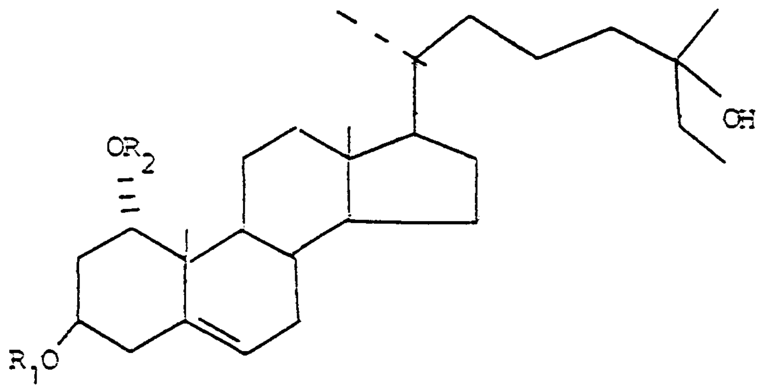

dans laquelle $R_1$ et $R_2$ représentent tous deux un groupe acétyle.

**11.** Composé conforme à l'une quelconque des revendications 1 à 6, utilisé pour déclencher et stimuler la différentiation cellulaire dans les cellules malignes.